# EUROPÄISCHE PATENTANMELDUNG

(11) **EP 2 612 846 A1**
(43) Veröffentlichungstag der Anmeldung: **10.07.2013**
(21) Anmeldenummer: 12150420.3
(22) Anmeldetag: 09.01.2012
(51) Int. Cl.: C07C 213/08, C07C 219/20

(54) **Beta-Aminosäureester und deren Verwendung**

(71) Anmelder: Bayer MaterialScience AG, 51373 Leverkusen (DE)
(72) Erfinder: Die Erfindernennung liegt noch nicht vor

(57) **Zusammenfassung**

Die vorliegende Erfindung betrifft einen beta-Aminosäureester, erhältlich durch Umsetzung eines Di-Acrylats der allgemeinen Formel (I) 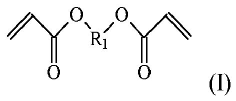 mit einem Diamin der allgemeinen Formel (II) 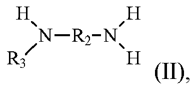 wobei R₁ und R₂ organische Reste ohne Zerewitinoff-aktive H-Atome darstellen und R₃ für einen organischen Rest ohne Zerewitinoff-aktives H-Atom oder ein Wasserstoffatom steht. Die Erfindung betrifft ferner ein Verfahren zur Herstellung eines solchen beta-Aminosäureesters und ein Polyhanstoffsystem, das einen derartigen beta-Aminosäureester enthält.

## Beschreibung

Die Erfindung betrifft einen Beta-Aminosäureester, insbesondere einen oligomeren oder polymeren Beta-Aminosäureester, ein Verfahren zu dessen Herstellung sowie die Verwendung dieser Verbindung als Härter zur Herstellung von Polyurethanharnstoffen oder Polyharnstoffen, insbesondere für Adhäsionsbarrieren und Gewebekleber.

Zu den häufigsten Komplikationen nach Eingriffen im Bauch- und Beckenbereich gehören Adhäsionen. Adhäsionen sind fibröse Bänder, die im Allgemeinen innerhalb der ersten sieben Tage nach einer Operation im Rahmen des Heilungsprozesses entstehen. Dadurch wachsen Gewebe und Organe, die normalerweise voneinander separiert sind, zusammen, wodurch eine Vielzahl von Komplikationen wie z.B. chronische Schmerzen, Unfruchtbarkeit oder ein lebensbedrohlicher Darmverschluss auftreten können. Zur Vermeidung derartiger Komplikationen wurden in den letzten Jahren Produkte entwickelt, die die Bildung von Adhäsionen verringern können. Der Erfolg war bislang jedoch limitiert.

Methoden zur Prävention von Adhäsionen sind das Auswaschen der peritonealen Kavität, die Anwendung pharmakologisch aktiver Agentien wie antiinflammatorisch oder fibrinolytisch wirksamer Verbindungen sowie die Applikation mechanisch wirkender Barrieren zur Trennung des Gewebes. Adhäsionsbarrieren bestehen aus einem inerten oder absorbierbaren Material, das auf die entsprechenden Organe aufgetragen wird. Untersucht wurde eine Vielzahl von Materialien wie Polysaccharide (US 4886787, US 5,356,883), Hyaluronsäure (US 4,141,973, US 5,246,698), Alginate (US 5,266,326), Chitin (US 5,093,319), Chitosan (US 4,532,134, US 5,679,658), Xanthan (US 4,994,277), Dextran (US 5,605,938), Cellulose und deren Derivate (Journal of spinal disorders & techniques (2006), 19(4), 270-5), humanes Serum Albumin (US 5,583,114), Collagen (US 2005/175659), Glucosamine (US 5,462,976), Polyoxyalkylencopolymere (US 4,911,926, US 5,366,735, US 5,135,751, US 5,681,576), Polyester (US 5,612,052, US 6,136,333) etc. Ein Großteil dieser Materialien konnte sich wegen mangelnder Wirksamkeit, fehlender Bioabbaubarkeit oder aufgrund von Interaktionen mit der Wundheilung nicht kommerziell durchsetzen.

Kommerziell erhältlich sind Produkte in Membranform wie INTERCEED™ (Johnson & Johnson), SEPRAFILM™ (Genzyme Corp.) und REPEL-CV™ (Life Medical Corp.), die innerhalb von 28 Tagen absorbiert werden. Da die Barrieren auf das entsprechende Organ aufgelegt werden, besteht jedoch die Gefahr des Verrutschens.

Barrieren, die wie die Hyaluronsäurederivate SEPRACOAT™ (GenzymeCorp.) und LUBRICOAT™ (Lifecore Biomedical Inc.) als Flüssigkeit appliziert werden, werden oft zu schnell vom Körper abgebaut, wodurch die Barrierewirkung eingeschränkt ist. Zusätzlich besteht die Gefahr der Migration, so dass die Schutzwirkung entfällt.

Weiterhin ist der Einsatz von Hydrogelen bekannt. Hydrogele sind wasserhaltige Polymere, deren Ketten kovalent zu einem dreidimensionalen Netzwerk verknüpft sind. In Wasser quellen sie schnell und unter starker Volumenzunahme. Aufgrund ihres hohen Wassergehaltes sind Hydrogele prinzipiell als Adhäsionsbarrieren interessant. Neben auf natürlichen Polysacchariden basierenden Hydrogelen (Alginate, Hyaluronsäure) sind insbesondere hydrophile Polyethylenglykol-basierte Systeme (US 2005/266086, DE 699 29 278, US 7,025,990, US 6,514,534, US 2003/0077242) intensiv erforscht worden, wie das kommerziell erhältliche SPRAYGEL™ (Confluent Surgical). Nachteilig erwiesen sich hierbei jedoch die mitunter zu hohe Abbaugeschwindigkeit und die Azidität der Abbauprodukte bei Verwendung von Milchsäure-basierten Polyestern.

Neben der Polyethylenglykol-basierten Hydrogelbildung findet die Redox-System-initiierte radikalische Polymerisation häufige Erwähnung (WO 00/09087, US 2003/0077242, US 2005/0271727). Als Redoxinitiatoren werden u.a. Ascorbinsäure und Peroxide eingesetzt. Neben möglichen Gewebeirritationen ergibt sich die Problematik der wässrigen Konsistenz der beiden Reaktionspartner, wodurch bei diesem System die Haftung auf menschlichem oder tierischem Organgewebe nachteilig beeinflusst wird.

In US 2004/0068078 und WO 2004/021983 werden mit Isocyanatgruppen gekappte Polymere wie Polyester- und Polyetherurethane als postoperative Adhäsionsbarrieren beschrieben. Als Isocyanate werden bevorzugt TDI und IPDI (Isophorondiisocyanat) eingesetzt, wobei die Präpolymere einen Gehalt niedrigmolekularer Polyisocyanate wie TDI von 0.05 bis 1 mEq enthalten, um eine gute Adhäsion am Gewebe zu bewirken. Bei Anwesenheit biologischer Flüssigkeiten oder bei bestimmten Gewebetypen sollen größere Mengen hiervon vorhanden sein. Die Adhäsion erfolgt unter anderem durch Reaktion des Isocyanates mit dem Gewebe. Monomere Isocyanate sind jedoch bekannt dafür, neben Gewebereizungen zu einer Sensibilisierung und damit zu allergischen Reaktionen zu führen. Die Reaktionsgeschwindigkeit des Präpolymers auf dem Gewebe ist bei Verwendung aliphatischer Isocyanate wie HDI zudem stark verlangsamt, so dass ein solches System für die klinische Anwendung nicht praktikabel ist.

In US 7,129,300 ist die Herstellung absorbierbarer 2-Komponentensysteme beschrieben, die aus einem Polyethylenoxid mit zwei oder mehr Amin-Substituenten und einem biologisch abbaubaren Diisocyanat beziehungsweise einem Isocyanat gekappten Polyethylenoxid mit einem absorbierbaren Diamin bestehen.

WO 2006/010278 beschreibt die Herstellung und Verwendung von Polyurethan-Präpolymeren und Polyurethanacrylaten als Adhäsionsbarriere. Diese Zusammensetzungen basieren auf aliphatischen Isocyanaten wie HDI als Kettenverlängerer beziehungsweise Härter werden niedermolekulare Diole, Diamine, Triole, Triamine oder Oligomere sowie physiologisch aktive Verbindungen vorgeschlagen. Zudem enthält die Zusammensetzung einen Katalysator, wofür organische Zink-, Zinn-, Magnesium- und Eisenverbindungen zum Einsatz kommen. Die Verwendung eines Katalysators führt jedoch im Allgemeinen zu einer starken Beschleunigung der Aushärtungsgeschwindigkeit des Polymers, was mit einer erhöhten Wärmeentwicklung einhergeht. Dadurch ist die Anwendung auf inneren Organen eingeschränkt.

In EP 1 719 530 A1 ist die Verwendung Isocyanat-gekappter Polyester-Makromere basierend auf aliphatischen Dicarbonsäuren und Dihydroxykomponenten wie Polyalkylenoxiden oder Polyethylenglykolen offenbart. Als mögliche Isocyanate werden aromatische, aliphatische und alicyclische Isocyanate genannt. Präpolymere aus Aromatenbasierten Isocyanaten wie TDI (wie in den Beispielen aufgeführt) haben eine angegebene Vernetzungszeit auf Gewebe von 1-10 min. Der Einsatz Aromaten-basierter Isocyanate ist für die Anwendung im Körper, bei der wie bei den Adhäsionsbarrieren das Produkt vollständig abgebaut wird, jedoch aufgrund der entstehenden Spaltprodukte als kritisch zu betrachten. Auf aliphatischen Isocyanaten basierte Systeme zeigen allerdings erfahrungsgemäß eine nur ungenügende Reaktivität und damit eine zu langsame Aushärtungsgeschwindigkeit, die für die Anwendung in vivo nicht praktikabel ist. Zusätzlich sind die Viskositäten der in EP 1 719 530 A1 aufgeführten Verbindungen mit im Durchschnitt 60.000 mPas zu hoch, um applizierbar zu sein, so dass ein Lösungsmittel verwendet werden muss, was unerwünscht ist.

Die WO 2007/067624 offenbart ein bioabsorbierbares 2-Komponentensystem, bestehend aus einem Isocyanatpräpolymer basierend auf Glycolid, Lactid, e-Caprolacton, p-Dioxanon, Trimethylcarbonat und Polyalkylenoxid (z.B. Polyethylenglycole). Als zweite Komponente wird ein Polyamin eingesetzt. Bei der Applikation beider Komponenten auf das Gewebe bildet sich ein Gel, welches als Klebstoff oder Adhäsionsbarriere eingesetzt werden kann. Die Präpolymere sind jedoch sehr hochviskos, so dass eine Applikation ohne Lösungsmittelzusatz nur schwer möglich sein dürfte. Als mögliche Lösungsmittel werden u.a. Wasser, Alkohole und Ketone angegeben. Hydroxylgruppenhaltige Lösungsmittel bergen jedoch das Problem einer schnellen Reaktion mit dem Präpolymer, so dass die Gefahr der Vergelung besteht. Die Verarbeitungszeit kann sich dadurch stark verkürzen wodurch die Anwendbarkeit leidet. Die Verwendung von Lösungsmitteln ist allgemein beim Einsatz in vivo in den meisten Fällen aufgrund von möglicher Cytotoxizität sowie Wechselwirkung mit dem Gewebe als kritisch einzustufen.

Die der vorliegenden Erfindung zugrunde liegende Aufgabe bestand nun darin, einen Härter zur Verfügung zu stellen, mit dem eine Adhäsionsbarriere bereitgestellt werden kann, welche einen flexiblen Film auf den zu schützenden Organen oder dem Gewebe bildet und eine gute Adhäsion auf den zu schützenden Organen oder dem Gewebe zeigt. Gleichzeitig soll der Härter eine hohe Aushärtegeschwindigkeit ermöglichen, die eine zügige Verarbeitbarkeit sicherstellt. Zudem soll das Material der Adhäsionsbarriere biokompatibel sein und möglichst binnen 6 Monaten oder weniger nach der Anwendung im menschlichen oder tierischen Körper biologisch abgebaut sein.

Diese Aufgabe wird gelöst durch einen beta-Aminosäureester erhältlich durch Umsetzung eines Di-Acrylats der allgemeinen Formel (I) 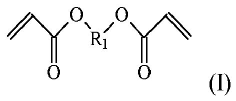 mit einem Diamin der allgemeinen Formel (II) 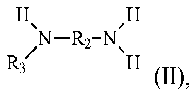 wobei R₁ und R₂ organische Reste ohne Zerewitinoff-aktive H-Atome darstellen und R₃ für einen organischen Rest ohne Zerewitinoff-aktives H-Atom oder ein Wasserstoffatom steht.

Überraschenderweise hat sich herausgestellt, dass bei Verwendung eines solchen beta-Aminosäureesters zur Härtung von NCO-terminierten Präpolymeren erzeugbare Adhäsionsbarrieren gut haftende und gleichzeitig flexible Filme auf den zu schützenden Organen bilden. Diese härten zudem in kürzester Zeit aus, sind also "tack-free". Ein möglicher Grund hierfür kann darin gesehen werden, dass die Additionsprodukte der Verbindungen der Formeln (I) und (II) polyvalente Vernetzermoleküle darstellen, die demzufolge die zur Aushärtung notwendige Perkolationsschwelle schneller erreichen und ein raumübergreifendes Netzwerk in kürzerer Zeit aufgebaut werden kann.

Die mit dem erfindungsgemäßen beta-Aminosäureester ausgehärteten Polyurethanpräpolymere zeichnen sich ferner durch eine hohe Biokompatibilität aus und werden in der Regel binnen weniger Wochen nach der Anwendung im menschlichen oder tierischen Körper abgebaut. Die dabei entstehenden Abbauprodukte besitzen zudem keine Zell- und Gewebetoxizität, was ebenfalls von Vorteil ist.

Trotz der hohen Aushärtegeschwindigkeit entwickelt ein mit dem erfindungsgemäßen beta-Aminosäureester ausgehärtetes NCO-terminiertes Präpolymere in wässriger Lösung bei der Aushärtung keine nennenswerte oder gar gewebeschädigende Exothermie.

Ein weiterer Vorteil des erfindungsgemäßen beta-Aminosäureesters besteht darin, dass dessen Viskosität variabel eingestellt werden kann, wodurch eine leichte Applizierbarkeit gewährleistet ist. Gleichzeitig wird ein Eindringen in tiefere Gewebeschichten vermieden. Dabei lässt sich die Viskosität vornehmlich über die Wahl der Reste R₁ und R₂ aber in gewissem Maße auch durch R₃ beeinflussen. Ebenso kann über die Kettenlänge des bei der Umsetzung der Verbindungen der Formeln (I) und (II) entstehenden oligomeren beziehungsweise polymeren beta-Aminosäureesters dessen Viskosität modifiziert werden.

Erfindungsgemäß weisen die Reste R₁, R₂ und R₃ keine Zerewitinoff-aktiven H-Atome. Unter einem Zerewitinoff-aktiven H-Atom wird im Rahmen der vorliegenden Erfindung ein azides H-Atom oder "aktives" H-Atom verstanden. Ein solches kann in an sich bekannter Weise durch eine Reaktivität mit einem entsprechenden Grignard-Reagenz ermittelt werden. Die Menge an Zerewitinoff-aktiven H-Atomen wird typischerweise über die Methanfreisetzung bestimmt, die bei einer Reaktion der zu überprüfenden Substanz mit Methylmagnesiumbromid (CH₃-MgBr) gemäß der folgenden Reaktionsgleichung (Formel 1) frei wird:

CH₃-MgBr + ROH → CH₄ + Mg (OR)Br (1)

Zerewitinoff-aktive H-Atome stammen typischerweise von C-H aziden organischen Gruppen, -OH, -SH, -NH₂ oder -NHR mit R als organischem Rest sowie -COOH.

Bei den erfindungsgemäßen beta-Aminosäureestern kann die Zahl der jeweils auf die Formeln (I) und (II) zurück gehenden Einheiten über weite Bereiche variieren. Beispielsweise können von den auf die Formeln (I) und (II) zurückgehenden Einheiten jeweils 2 bis 20 Einheiten in dem beta-Aminosäureester vorhanden sein, wobei die Anzahl der auf die Formel (I) und (II) zurückgehenden Einheiten gleich sein oder sich um jeweils eine Einheit unterscheiden kann, je nachdem, mit welcher der Einheiten der beta-Aminosäureester terminiert ist.

In der Formel (II) ist wenigstens eine der Aminogruppen eine primäre Aminogruppe. Bevorzugt sind jedoch beide Aminogruppen primäre Aminogruppen. Der Grund hierfür ist, dass bei der Addition einer sekundären Aminogruppe an die acrylische Doppelbindung ein tertiäres Amin entsteht, welches folglich kein freies Wasserstoffatom mehr zur Reaktion mit der NCO-Gruppe eines Polyisocyanats besitzt. Durch die Verwendung eines Diamins nach Formel (II) mit einer sekundären Aminogruppe wird also die Zahl der reaktiven Zentren pro Einheit der Formel (II) im beta-Aminosäureester reduziert.

Dennoch kann in bestimmten Fällen der zumindest teilweise Einsatz von Aminen mit einer primären und einer sekundären Aminigruppe gemäß Formel (II) gewünscht sein. So können die bei der Umsetzung mit Verbindungen der Formel (I) entstehenden tertiären Aminogruppen bei der Aushärtung von NCO-Präpolymeren katalysierend wirken. Zudem kann bei vergleichsweise kurzkettigen Resten R₁ und R₂ der Abstand der NCO-reaktiven Gruppen erhöht werden, wodurch ein weicheres Polymer erhalten werden kann.

Für die Reste R₁ und R₂ kommen eine Vielzahl von organischen Resten in Frage. In vorteilhafter Ausgestaltung der erfindungsgemäßen beta-Aminosäureester sind R₁ und R₂ unabhängig voneinander ausgewählt aus Polyesterpolyolgruppen, Polyester-Polyether-Polyolgruppen und/oder Polyetherpolyolgruppen. Diese Gruppen zeichnen sich durch eine vergleichsweise hohe Polarität aus, wodurch eine bessere Gewebeadhäsion erzielt wird. Zudem sind diese Reste leichter biologisch abbaubar. Auch sind diese Bausteine flexibel, was sich bei der Umsetzung mit Polyisocyanaten vorteilhaft auf die Weichheit der dabei entstehenden Polyhamstoff-Polymere auswirkt. Besonders bevorzugt sind hierbei Polyester-Polyether-Polyolgruppen und/oder Polyetherpolyolgruppen mit einem Ethylenoxidanteil von wenigstens 60 Gew.-%.

Was die Kettenlänge beziehungsweise das Molekulargewicht der Reste R₁ und R₂ im erfindungsgemäßen beta-Aminosäureester betrifft, so können diese über weite Bereiche variieren. Hierbei ist es bevorzugt, wenn die Reste R₁ und R₂ unabhängig voneinander ein Gewichtsmittel von 200 bis 2000 g/mol aufweisen, insbesondere 300 bis 1500 g/mol, bevorzugt 400 bis 1000 g/mol. Dies ist besonders vorteilhaft, weil dadurch eine gute Adhäsion am Gewebe erreicht wird. Zudem werden hierdurch bei der Umsetzung mit Polyisocyanaten dreidimensionale Polymernetzwerke geschaffen, die zwischen den einzelnen polymeren Ästen große und durch hydrophile Gruppen der Reste R₁ und R₂ und des Polymerbackbones der Polyisocyanate eingefasste Zwischenräume aufweisen. In diese Zwischenräume kann eine größere Menge Wasser eingelagert werden. Hierdurch besitzen die bei der Umsetzung entstandenen Polyharnstoff-Polymere ein hohes Quell- und Wasseraufnahmevermögen und sind bestens zur Bildung von Hydrogelen geeignet.

Bei dem erfindungsgemäßen beta-Aminosäureester kann der Rest R₃ aus einer Vielzahl von Verbindungen ausgewählt sein. Wie bereits vorstehend ausgeführt, ist ein Wasserstoffrest besonders bevorzugt. Zudem kann R₃ auch aus gesättigten oder ungesättigten Kohlenwasserstoffresten ausgewählt sein, insbesondere aus Methyl-, Ethyl- oder Propylresten. Hierbei sind kurzkettige aliphatische Reste mit bis zu 3 Kohlenstoffatomen besonders bevorzugt, da von diesen eine geringere sterische Hinderung ausgeht.

Wie bereits vorstehend erwähnt, kann der erfindungsgemäße beta-Aminosäureester eine oligomere oder polymere Struktur aufweisen, die in der Regel aus einer alternierenden Anordnung der Strukturelemente der Formeln (I) und (II) besteht. Was die terminalen Gruppen angeht, existieren mehrere Alternativen zum Aufbau des beta-Aminosäureesters. So kann beispielsweise bei Einsatz eines primären Diamins nach Formel (II) der beta-Aminosäureester der allgemeinen Formel (III) entsprechen 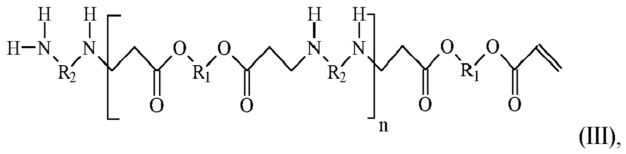 wobei n eine ganze Zahl darstellt, die insbesondere bei 1 bis 20 liegt.

Mit anderen Worten bildet hierbei jeweils eine primäre Aminogruppe der Verbindung nach Formel (II) das eine Kettenende und eine acrylische Doppelbindung des Diacrylats gemäß Formel (I) das andere Kettenende. Gleichermaßen kann der beta-Aminosäureester jeweils zwei (primäre) Amine an den jeweiligen Kettenenden aufweisen oder aber auch zwei acrylische Doppelbindungen. In der Regel wird sich eine Mischung der vorgenannten Verbidnungen einstellen, sofern nicht eine Verbindung der Formel (I) oder (II) im entsprechenden Überschuss eingesetzt wird. Vorzugsweise werden die Verbindungen der Formel (I) und (II) jedoch in einem Äquivalentverhältnis von etwa 0,6 : 1 bis 1,5 : 1 eingesetzt, insbesondere von etwa 0,7 : 1 bis 1,1 : 1. Vorzugsweise liegt das Verhältnis bei etwa 0, 8 : 1.

Selbiges gilt auch für den Einsatz eines Diamins nach Formel (II) mit einer sekundären Aminogruppe, wobei hier noch wegen der fehlenden Symmetrie die Möglichkeit besteht, dass das Kettenende durch die primäre oder durch die sekundäre Aminogruppe definiert ist.

Das Molekulargewicht des erfindungsgemäßen beta-Aminosäureesters kann über weite Bereiche angepasst werden. Vorzugsweise weist der beta-Aminosäureester ein zahlenmittleres Molekulargewicht von 1300 bis 25000 auf, insbesondere von 3000 bis 12000.

Ein weiterer Gegenstand der vorliegenden Erfindung betrifft ein Verfahren zur Herstellung eines erfindungsgemäßen beta-Aminosäureesters bei dem man ein Di-Acrylat der allgemeinen Formel (I) mit einem Diamin der allgemeinen Formel (II) umsetzt. Hierbei kann im einfachsten Fall so vorgegangen werden, dass das Diamin zunächst vorgelegt und das Diacrylat unter Rühren hinzugetropft wird. Die Temperatur sollte vorzugsweise nicht über 30 °C steigen. Da die vorgenannte Umsetzung stark exotherm verläuft, ist eine Kühlung des Reaktionsgefäßes ratsam. Nach beendeter Zugabe und abgeklungener Exothermie kann anschließend auf eine Temperatur von beispielsweise 60 °C erhitzt werden. Diese Temperatur wird vorzugsweise für 10 bis 14 Stunden gehalten.

Ein weiterer Gegenstand der vorliegenden Erfindung betrifft ein Polyharnstoff System, das die folgenden Komponenten umfasst:
als Komponente A) isocyanatfunktionelle Präpolymere erhältlich durch Umsetzung von
aliphatischen Polyisocyanaten A1) mit
Polyolen A2), die insbesondere ein zahlenmittleres Molekulargewicht von ≥ 400 g/mol und eine mittlere OH-Funktionalität von 2 bis 6 aufweisen können,
als Komponente B) ein erfindungsgemäßer beta-Aminosäureester,
gegebenenfalls als Komponente C) organische Füllstoffe, die insbesondere eine nach DIN 53019 gemessenen Viskosität bei 23 °C im Bereich von 10 bis 6000 mPas aufweisen können,
gegebenenfalls als Komponente D) Umsetzungsprodukte von isocyanatfunktionellen Präpolymeren gemäß Komponente A) mit Verbindungen gemäß Komponente B) und/oder organischen Füllstoffen gemäß Komponente C) und
gegebenenfalls als Komponente E) Wasser und/oder ein tertiäres Amin.

Die erfindungsgemäßen Polyhamstoff-Systeme werden durch Mischung der Präpolymere A) mit dem erfindungsgemäßen beta-Aminosäureester B) sowie gegebenenfalls den Komponenten C), D) und/oder E) erhalten. Das Verhältnis von freien oder blockierten Aminogruppen zu freien NCO-Gruppen beträgt dabei bevorzugt 1:1,5, besonders bevorzugt 1:1. Wasser und/oder Amin werden dabei der Komponente B) bzw. C) beigemischt.

Die isocyanatfunktionellen Präpolymere A) sind durch Umsetzung von Polyisocyanaten A1) mit Polyolen A2) gegebenenfalls unter Zusatz von Katalysatoren sowie Hilfs- und Zusatzstoffen erhältlich.

Als Polyisocyanate A1) können beispielsweise monomere aliphatische oder cycloaliphatische Di- oder Triisocyanate wie 1,4-Butylendiisocyanat (BDI), 1,6-Hexamethylendiisocyanat (HDI), Isophorondiisocyanat (IPDI), 2,2,4- und/oder 2,4,4-Trimethylhexa-methylendiisocyanat, die isomeren Bis-(4,4'-isocyanatocyclohexyl)-methane oder deren Mischungen beliebigen Isomerengehalts, 1,4-Cyclohexylendiisocyanat, 4-Isocyanatomethyl-l,8-octandiisocyanat (Nonan-triisocyanat), sowie Alkyl-2,6-diisocyanatohexanoat (Lysindiisocyanat) mit C1-C8-Alkylgruppen eingesetzt werden.

Neben den vorstehend genannten monomeren Polyisocyanaten A1) können auch deren höhermolekulare Folgeprodukte mit Uretdion-, Isocyanurat-, Urethan-, Allophanat-, Biuret-, Iminooxadiazindion- oder Oxadiazintrionstruktur sowie deren Mischungen eingesetzt werden.

Bevorzugt werden Polyisocyanate A1) der vorstehend genannten Art mit ausschließlich aliphatisch oder cycloaliphatisch gebundenen Isocyanatgruppen oder deren Mischungen eingesetzt.

Ebenfalls bevorzugt ist, wenn Polyisocyanate A1) der vorstehenden Art mit einer mittlere NCO-Funktionalität von 1,5 bis 2,5, bevorzugt von 1,6 bis 2,4, weiter bevorzugt von 1,7 bis 2,3, ganz besonders bevorzugt von 1,8 bis 2,2 und insbesondere von 2 verwendet werde.

Ganz besonders bevorzugt wird Hexamethylendiisocyanat als Polyisocyanat A1) eingesetzt. Gemäß einer bevorzugten Ausführungsform des erfindungsgemäßen Polyharnstoff Systems ist vorgesehen, dass die Polyole A2) Polyesterpolyole und/oder Polyester-Polyether-Polyole und/oder Polyetherpolyole sind. Insbesondere bevorzugt sind dabei Polyester-Polyether-Polyole und/oder Polyetherpolyole mit einem Ethylenoxidanteil zwischen 60 und 90 Gew.-%.

Bevorzugt ist auch, wenn die Polyole A2) ein zahlenmittleres Molekulargewicht von 4000 bis 8500 g/mol aufweisen.

Geeignete Polyetheresterpolyole werden entsprechend dem Stand der Technik vorzugsweise durch Polykondensation aus Polycarbonsäuren, Anhydriden von Polycarbonsäuren, sowie Estern von Polycarbonsäuren mit leichtflüchtigen Alkoholen, bevorzugt C1 bis C6 Monoolen, wie Methanol, Ethanol, Propanol oder Butanol, mit molar überschüssigem, niedermolekularem und/oder höhermolekularem Polyol hergestellt; wobei als Polyol ethergruppenhaltige Polyole gegebenenfalls in Mischungen mit anderen ethergruppenfreien Polyolen eingesetzt werden.

Selbstverständlich können zur Polyetherestersynthese auch Gemische der höhermolekularen und der niedermolekularen Polyole verwendet werden.

Solche molar überschüssigen niedermolekularen Polyole sind Polyole mit Molmassen von 62 bis 299 Dalton, mit 2 bis 12 C-Atomen und Hydroxylfunktionalitäten von mindestens 2, die weiterhin verzweigt oder unverzweigt sein können und deren Hydroxylgruppen primär oder sekundär sind. Diese niedermolekularen Polyole können auch Ethergruppen aufweisen. Typische Vertreter sind Ethylenglykol, Propandiol-1,2, Propandiol-1,3, Butandiol-1,4, Butandiol-2,3, 2-Methyl-propandiol-1,3, Pentandiol-1,5, Hexandiol-1,6, 3-Methylpentandiol-1,5, 1,8-Octandiol, 1,10-Decandiol, 1,12-Dodecandiol, Cyclohexandiol, Diethylenglykol, Triethylenglykol und höhere Homologe, Dipropylenglykol, Tripropylenglykol und höhere Homologe, Glycerin, 1,1,1-Trimethylolpropan, sowie Oligotetrahydrofurane mit Hydroxylendgruppen. Selbstverständlich können innerhalb dieser Gruppe auch Gemische verwendet werden.

Molar überschüssige höhermolekulare Polyole sind Polyole mit Molmassen von 300 bis 3000 Dalton, die durch ringöffenende Polymerisation von Epoxiden, bevorzugt Ethylen- und/oder Propylenoxid, sowie durch säurekatalysierte, ringöffnende Polymerisation von Tetrahydrofuran, erhalten werden können. Zur ringöffnenden Polymerisation von Epoxiden können entweder Alkalihydroxide oder Doppelmetallcyanidkatalysatoren verwendet werden. Als Starter für ringöffnende Epoxidpolymerisationen können alle mindestens bifunktionellen Moleküle aus der Gruppe der Amine und der o.g. niedermolekularen Polyole verwendet werden. Typische Vertreter sind 1,1,1-Trimethylolpropan, Glycerin, o-TDA, Ethylendiamin, Propylenglykol-1,2, etc. sowie Wasser, einschließlich deren Gemische. Selbstverständlich können innerhalb der Gruppe der überschüssigen höhermolekularen Polyole auch Gemische verwendet werden.

Der Aufbau der höhermolekularen Polyole, soweit es sich um Hydroxylgruppen terminierte Polyalkylenoxide aus Ethylen- und/oder Propylenoxid handelt, kann statistisch oder blockweise erfolgen, wobei auch Mischblöcke enthalten sein können.

Polycarbonsäuren sind sowohl aliphatische als auch aromatische Carbonsäuren, die sowohl cyclisch, linear, verzweigt oder unverzweigt sein können und die zwischen 4 und 24 C-Atome aufweisen können.

Beispiele sind Bernsteinsäure, Glutarsäure, Adipinsäure, Azelainsäure, Sebacinsäure, 1,10-Decandicarbonsäure, 1,12-Dodecandicarbonsäure, Phthalsäure, Terephthalsäure, Isophthalsäure, Trimellitsäure, Pyromellitsäure. Bevorzugt sind Bernsteinsäure, Glutarsäure, Adipinsäure, Sebacinsäure, Milchsäure, Phthalsäure, Terephthalsäure, Isophthalsäure, Trimellitsäure, Pyromellitsäure. Besonders bevorzugt sind Bernsteinsäure, Glutarsäure und Adipinsäure.

Weiterhin umfasst die Gruppe der Polycarbonsäuren auch Hydroxycarbonsäuren, bzw. deren innere Anhydride, wie z.B. Caprolacton, Milchsäure, Hydroxybuttersäure, Ricinolsäure, usw. Mit umfasst sind weiterhin auch Monocarbonsäuren, insbesondere solche, die über mehr als 10 C-Atome verfügen, wie Sojaölfettsäure, Palmölfettsäure und Erdnussölfettsäure, wobei deren Anteil am gesamten, das Polyetheresterpolyol aufbauenden Reaktionsmischung 10 Gew.-% nicht übersteigt und zusätzlich die dadurch einhergehende Minderfunktionalität durch Mitverwendung von mindestens trifunktionellen Polyolen, sei es auf Seiten der niedermolekularen oder der hochmolekularen Polyole ausgeglichen wird.

Die Herstellung der Polyetheresterpolyol erfolgt entsprechend dem Stand der Technik bei erhöhter Temperatur im Bereich von 120 bis 250 °C, zunächst unter Normaldruck, später unter Anlegen von Vakuum von 1 bis 100 mbar, vorzugsweise, aber nicht notwendigerweise unter Verwendung eines Veresterungs- oder Umesterungskatalysators, wobei die Reaktion so weit vervollständigt wird, dass die Säurezahl auf Werte von 0,05 bis 10 mg KOH/g, bevorzugt 0,1 bis 3 mg KOH/g und besonders bevorzugt 0,15 bis 2,5 mg KOH/g absinkt. Weiterhin kann im Rahmen der Normaldruckphase vor dem Anlegen von Vakuum ein Inertgas verwendet werden. Selbstverständlich können alternativ oder für einzelne Phasen der Veresterung auch flüssige oder gasförmige Schleppmittel zum Einsatz kommen. Beispielsweise kann das Reaktionswasser unter Verwendung von Stickstoff als Trägergas, ebenso ausgetragen werden, wie unter Einsatz eines Azeotropschleppmittels, wie z.B. Benzol, Toluol, Xylol, Dioxan, etc.

Selbstverständlich können auch Abmischungen von Polyetherpolyolen mit Polyesterpolyolen in beliebigen Verhältnissen eingesetzt werden.

Polyetherpolyole sind bevorzugt Polyalkylenoxid-Polyether auf Basis von Ethylenoxid und gegebenenfalls Propylenoxid.

Diese Polyetherpolyole basieren bevorzugt auf di- oder höherfunktionellen Startermolekülen wie zwei- oder höherfunktionellen Alkoholen oder Aminen.

Beispiele solcher Starter sind Wasser (als Diol aufgefasst), Ethylenglykol, Propylenglykol, Butylenglykol, Glycerin, TMP, Sorbit, Pentaerythrit, Triethanolamin, Ammoniak oder Ethylendiamin.

Ebenfalls können Hydroxylgruppen aufweisende Polycarbonate, bevorzugt Polycarbonatdiole, mit zahlenmittleren Molekulargewichten Mn von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylen-glykol, Polybutylenglykole, Bisphenol A und lactonmodifizierte Diole der vorstehend genannten Art in Frage.

Zur Herstellung des Präpolymers A) kann das Polyisocyanat A1) mit dem Polyol A2) bei einem NCO/OH-Verhältnis von bevorzugt 4:1 bis 12:1, besonders bevorzugt 8:1 umgesetzt und anschließend der Anteil an nicht umgesetzten Polyisocyanates mittels geeigneter Methoden abgetrennt werden. Üblicherweise wird hierfür die Dünnschichtdestillation verwendet, wobei Präpolymere mit Restmonomergehalten von weniger als 1 Gew.-%, bevorzugt weniger als 0,1 Gew.-%, ganz besonders bevorzugt weniger als 0,03 Gew.-% erhalten werden.

Gegebenenfalls können während der Herstellung Stabilisatoren wie Benzoylchlorid, Isophthaloylchlorid, Dibutylphosphat, 3-Chlorpropionsäure oder Methyltosylat zugesetzt werden.

Die Reaktionstemperatur bei der Herstellung der Präpolymere A) beträgt dabei bevorzugt 20 bis 120 °C und weiter bevorzugt 60 bis 100 °C.

Die hergestellten Präpolymere haben einen nach DIN EN ISO 11909 gemessenen mittleren NCO-Gehalt von 2 bis 10 Gew.-%, bevorzugt 2,5 bis 8 Gew.-%.

Gemäß einer weiteren Ausführungsform des erfindungsgemäßen Polyharnstoff Systems können die Präpolymere A) eine mittlere NCO-Funktionalität von 1,5 bis 2,5, bevorzugt von 1,6 bis 2,4, weiter bevorzugt von 1,7 bis 2,3, ganz besonders bevorzugt von 1,8 bis 2,2 und insbesondere von 2 aufweisen.

Bei den organischen Füllstoffen der Komponente C) kann es sich bevorzugt um hydroxyfunktionelle Verbindungen, insbesondere um Polyetherpolyole mit sich wiederholenden Ethylenoxid Einheiten handelt.

Vorteilhaft ist auch, wenn die Füllstoffe der Komponente C) eine mittlere OH-Funktionalität von 1,5 bis 3, bevorzugt von 1,8 bis 2,2 und besonders bevorzugt von 2 aufweisen.

Beispielsweise können als organische Füllstoffe bei 23 °C flüssige Polyethylenglykole wie PEG 200 bis PEG 600, deren Mono- bzw. Dialkylether wie PEG 500 Dimethylether, flüssige Polyether- und Polyesterpolyole, flüssige Polyester wie z.B. Ultramoll (Lanxess AG, Leverkusen, DE) sowie Glycerin und seine flüssigen Derivate wie z.B. Triacetin (Lanxess AG, Leverkusen, DE) eingesetzt werden.

Die Viskosität der organischen Füllstoffe gemessen nach DIN 53019 bei 23 °C beträgt bevorzugt 50 bis 4000 mPas, besonders bevorzugt 50 bis 2000 mPas.

In einer bevorzugten Ausführungsform des erfindungsgemäßen Polyharnstoff Systems werden als organische Füllstoffe Polyethylenglykole eingesetzt. Diese haben bevorzugt ein zahlenmittleres Molekulargewicht von 100 bis 1000 g/mol, besonders bevorzugt 200 bis 400 g/mol.

Um das mittlere Äquivalentgewicht der insgesamt zur Präpolymervernetzung eingesetzten Verbindungen bezogen auf die NCO-reaktiven Gruppen weiter zu reduzieren, ist es möglich zusätzlich Umsetzungsprodukte der Präpolymere A) mit der erfindungsgemäßen Verbindung als Komponente B) und/oder den organischen Füllstoffen C), sofern diese amino- oder hydroxyfunktionell sind, in einer separaten Vorreaktion herzustellen und dann als höhermolekulare Härterkomponente einzusetzen.

Bevorzugt werden bei der Vorverlängerung Verhältnisse von isocyanatreaktiven Gruppen zu Isocyanatgruppen von 50 zu 1 bis 1,5 zu 1, besonders bevorzugt 15 zu 1 bis 4 zu 1 eingestellt.

Vorteil dieser Modifizierung durch Vorverlängerung ist, dass das Äquivalentgewicht und das Äquivalentvolumen der Härterkomponente in größeren Grenzen modifizierbar ist. Dadurch können zur Applikation kommerziell verfügbare 2-Kammerdosiersysteme eingesetzt werden, um ein Klebesystem zu erhalten, das bei bestehenden Verhältnissen der Kammervolumina in das gewünschte Verhältnis von NCO-reaktiven Gruppen zu NCO-Gruppen eingestellt werden kann.

Gemäß einer weiteren bevorzugten Ausführungsform des erfindungsgemäßen Polyharnstoff-Systems ist vorgesehen, dass die Komponente E) ein tertiäre Amine der allgemeinen Formel (IV) 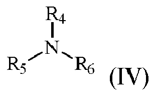 enthält, in der
R₄, R₅, R₆ unabhängig voneinander Alkyl- oder Heteroalkyreste mit Heteroatomen in der Alkylkette oder an deren Ende sein können, oder R₄ und R₅ gemeinsam mit dem sie tragenden Stickstoffatom einen aliphatischen, ungesättigten oder aromatischen Heterozyklus bilden können, der gegebenenfalls weitere Heteroatome enthalten kann.

Diese Polyharnstoff Systeme zeichnen sich durch eine besonders schnelle Aushärtung aus.

Bei den in Komponente E) verwendeten Verbindungen kann es sich ganz besonders bevorzugt um tertiäre Amine ausgewählt aus der Gruppe Triethanolamin, Tetrakis (2-hydroxyethyl) ethylendiamin, N,N-Dimethyl-2-(4-methylpiperazin-1-yl)ethanamin, 2-{[2-(Dimethylamino)ethyl](methyl)amino}ethanol, 3,3',3"-(1,3,5-Triazinan-1,3,5-triyl)tris(N,N-dimethyl-propan-1-amin) handeln.

Ganz besonders hohe Aushärtungsgeschwindigkeiten können auch erzielt werden, wenn die Komponente E) 0,2 bis 90 Gew.-% Wasser und/oder 0,1 bis 1,0 Gew.-% des tertiären Amins, jeweils bezogen auf das Polyharnstoff System, enthält.

Insbesondere dann, wenn aus dem erfindungsgemäßen Polyharnstoff System ein Hydrogel erzeugt werden soll, enthält das Polyharnstoff System vorzugsweise 10 bis 90 Gew.-% an Wasser, bezogen auf das Polyharnstoff-System.

Soll das erfindungsgemäße Polyhamstoff-System hingegen als Gewebeklebstoff verwendet werden ist es bevorzugt, wenn die Komponente E) 0,2 bis 2,0 Gew.-% Wasser bezogen auf das Polyharnstoff System enthält.

Selbstverständlich können in die Polyharnstoff Systeme auch pharmakologisch aktive Wirkstoffe wie Analgetika mit und ohne antiinflammatorische Wirkung, Antiphlogistika, antimikrobiell wirksame Substanzen, Antimykotika, antiparasitär wirkende Stoffe oder Kombinationen hiervon eingearbeitet werden.

Die Wirkstoffe können als reiner Wirkstoff oder aber in verkapselter Form vorliegen, um beispielsweise eine zeitlich verzögerte Abgabe zu erzielen. Als medizinische Wirkstoffe können im Rahmen der vorliegenden Erfindung eine Vielzahl von Wirkstofftypen und -klassen eingesetzt werden.

Ein solcher medizinischer Wirkstoff kann beispielsweise eine unter in vivo-Bedingungen Stickstoffmonoxid-freisetzende Komponente, bevorzugt L-Arginin oder eine L-Argininhaltige oder eine L-Arginin freisetzende Komponente, besonders bevorzugt L-Arginin Hydrochlorid umfassen. Auch Prolin, Ornithin und/oder andere biogene Zwischenstufen wie beispielsweise biogene Polyamine (Spermin, Spermitin, Putrescin oder bioaktive künstliche Polyamine) können verwendet werden. Derartige Komponenten unterstützen bekanntermaßen die Wundheilung, wobei deren kontinuierliche mengenmäßig nahezu gleichmäßige Abgabe der Wundheilung besonders zuträglich ist.

Weitere erfindungsgemäß verwendbare Wirkstoffe umfassen mindestens eine Substanz ausgewählt aus der Gruppe der Vitamine oder Provitamine, Carotinoide, Analgetika, Antiseptika, Hämostyptika, Antihistaminika, antimikrobiellen Metalle oder deren Salze, pflanzlichen wundheilungsfördernden Substanzen oder Substanzgemische, Pflanzenextrakte, Enzyme, Wachstumsfaktoren, Enzyminhibitoren sowie Kombinationen hiervon.

Als Analgetika sind insbesondere nicht-steroidale Analgetika insbesondere Salicylsäure, Acetylsalicylsäure und deren Derivate z.B. Aspirin®, Anilin und dessen Derivate, Acetaminophen z.B. Paracetamol®, Antranilsäure und deren Derivate z.B. Mefenaminsäure, Pyrazol oder dessen Derivate z.B. Methamizol, Novalgin®, Phenazon, Antipyrin®, Isopropylphenazon und ganz besonders bevorzugt Arylessigsäuren sowie deren Derivate, Heteroarylessigsäuren sowie deren Derivate, Arylpropionsäuren sowie deren Derivate und Herteroarylpropionsäuren sowie deren Derivate z.B. Indometacin®, Diclophenac®, Ibuprofen®, Naxoprophen®, Indomethacin®, Ketoprofen®, Piroxicam® geeignet.

Als Wachstumsfaktoren sind insbesondere zu nennen: aFGF (Acidic Fibroplast Growth Factor), EGF (Epidermal) Growth Factor), PDGF (Platelet Derived Growth Factor), rhPDGF-BB (Becaplermin), PDECGF (Platelet Derived Endothelial Cell Growth Factor), bFGF (Basic Fibroplast Growth Factor), TGF α; (Transforming Growth Factor alpha), TGF ß (Transforming Growth Factor beta), KGF (Keratinocyte Growth Factor), IGF1/IGF2 (Insulin-Like Growth Factor) und TNF (Tumor Necrosis Factor).

Als Vitamine oder Provitamine sind insbesondere die fettlöslichen oder wasserlöslichen Vitamine Vitamin A, Gruppe der Retinoide, Provitamin A, Gruppe der Carotenoide, insbesondere β-Carotin, Vitamin E, Gruppe der Tocopherole, insbesondere α Tocopherol, β-Tocopherol, γ-Tocopherol, δ-Tocopherol und α-Tocotrienol, β-Tocotrienol, γ-Tocotrienol und δ-Tocotrienol, Vitamin K, Phyllochinon insbesondere Phytomenadion oder pflanzliches Vitamin K, Vitamin C, L-Ascorbinsäure, Vitamin B1, Thiamin, Vitamin B2, Riboflavin, Vitamin G, Vitamin B3, Niacin, Nikotinsäure und Nikotinsäureamid, Vitamin B5, Pantothensäure, Provitamin B5, Panthenol oder Dexpanthenol, Vitamin B6, Vitamin B7, Vitamin H, Biotin, Vitamin B9, Folsäure sowie Kombinationen hiervon geeignet.

Als Antiseptikum ist ein solches Mittel zu verwenden, das gemizid, bakterizid, bakteriostatisch, fungizid, viruzid, virustatisch und/ oder allgemein mikrobiozid wirkt.

Insbesondere sind solche Stoffe geeignet die ausgewählt werden aus der Gruppe Resorcinol, Iod, Iod-Povidon, Chlorhexidin, Benzalkoniumchlorid, Benzoesäure, Benzoylperoxid oder Cethylpyridiniumchlorid. Darüber hinaus sind als Antiseptika insbesondere auch antimikrobiellen Metalle zu verwenden. Als antimikrobielle Metalle können insbesondere Silber, Kupfer oder Zink sowie deren Salze, Oxide oder Komplexe in Kombination oder alleine verwendet werden.

Als pflanzliche, wundheilungsfördernde Wirkstoffe sind im Zusammenhang mit der vorliegenden Erfindung insbesondere Extrakte der Kamille, Hamamelis-Extrakte z.B. Hamamelis virgina, Calendula-Extrakt, Aloe- Extrakt z.B. Aloe vera, Aloe barbadensis, Aloe feroxoder oder Aloe vulgaris, Grüntee- Extrakte, Meeresalgen-Extrakt z.B. Rotalgen- oder Grünalgen-Extrakt, Avocado-Extrakt, Myrre-Extrakt z.B. Commophora molmol, Bambus-Extrakte sowie Kombinationen hiervon zu nennen.

Der Gehalt der Wirkstoffe richtet sich dabei in erster Linie an der medizinisch erforderlichen Dosis aus sowie auch an der Verträglichkeit mit den übrigen Bestandteilen der erfindungsgemäßen Zusammensetzung.

Das erfindungsgemäße Polyharnstoff System ist besonders zum Verschließen, Verbinden, Verkleben oder Abdecken von Zellgewebe und insbesondere zur Erzeugung einer Adhäsionsbarriere und/ oder zur Stillung des Austritts von Blut oder Gewebeflüssigkeiten oder dem Verschluss von Leckagen in Zellgewebe geeignet. Ganz besonders bevorzugt kann es zur Verwendung oder zur Herstellung eines Mittels zum Verschließen, Verbinden, Verkleben oder Abdecken von menschliches oder tierisches Zellgewebe eingesetzt werden. Mit seiner Hilfe können schnell aushärtende, stark am Gewebe anhaftende, transparente, flexible und biokompatible Klebnähte hergestellt werden. Ebenso lassen sich damit schnell aushärtende Adhäsionsbarrieren erzeugen, die nach kurzer Zeit "tack-free" sind und sich postoperativ teilweise binnen weniger Tage oder Wochen abbauen.

Noch ein weiterer Gegenstand der Erfindung ist Dosiersystem mit zwei Kammern für ein erfindungsgemäßes Polyharnstoff System zur Erzeugung einer Adhäsionsbarriere, bei dem in der einen Kammer die Komponente A) eine erste Teilmenge von E) und in der anderen Kammer die Komponenten B) und gegebenenfalls die Komponenten C), D) und eine zweite Teilmenge von E) des Polyharnstoff Systems enthalten sind. Ein derartiges Dosiersystem eignet sich insbesondere dafür das Polyharnstoff System als Adhäsionsbarriere auf Gewebe zu applizieren. In diesem Fall enthält die Komponente E) bevorzugt 10 bis 90 Gew.-% Wasser, bezogen auf das Polyharnstoff-System.

Bei einem solchen Dosiersystem für eine Adhäsionsbarriere sind in vorteilhafter Weise die Komponenten A) und B) in zueinander äquivalenten Stoffmengen vorhanden und die erste und zweite Teilmenge von E) jeweils so gewählt, dass das Volumen von A) und erster Teilmenge von E) weitestgehend identisch zum Volumen aus B), der zweiten Teilmenge von E) und den optionalen Komponenten C) und D) ist. Eine solche Aufteilung ist vorteilhaft, weil sich dann mit dem Dosiersystem bei gleichen Volumenströmen aus den beiden Kammern ein Hydrogel ausbringen lässt, welches in kurzer Zeit abbindet und dabei eine zuverlässig wirkende Adhäsionsbarriere ausbildet.

Weiterhin betrifft die vorliegende Erfindung ein Dosiersystem mit zwei Kammern für ein erfindungsgemäßes Polyharnstoff System zur Verwendung als Gewebeklebstoff, bei dem in der einen Kammer die Komponente A) und in der anderen Kammer die Komponenten B) und gegebenenfalls die Komponenten C), D) und E) des Polyharnstoff-Systems enthalten sind. Ein derartiges Dosiersystem eignet sich insbesondere dafür das Polyharnstoff System als Gewebeklebstoff auf Gewebe zu applizieren. In diesem Fall enthält die Komponente E) bevorzugt 0,2 bis 2,0 Gew.-% Wasser, bezogen auf das Polyharnstoff System.

### Beispiele:

Die vorliegende Erfindung wird im Folgenden anhand von Ausführungsbeispielen näher erörtert.

### Methoden:

### Molekulargewicht:

Die Molekulargewichte wurden mittels Gelpermeationschromatographie (GPC) wie folgt bestimmt: Die Kalibrierung erfolgt mit Polystyrol-Standards mit Molekulargewichten von Mp 1.000.000 bis 162. Als Eluent wurde Tetrahydrofuran p.A. verwendet. Die folgenden Parameter wurden bei der Doppelmessung eingehalten: Entgasung: Online - Degasser; Durchfluß: 1 ml/Min, Analysenzeit: 45 Minuten; Detektoren: Refraktometer und UV-Detektor; Injektionsvolumen: 100 µl - 200 µl. Die Berechnung der Molmassenmittelwerte Mw; Mn und Mp sowie der Polydispersität Mw/Mn erfolgte softwaregestützt. Basislinienpunkte und Auswertegrenzen wurden entsprechend der DIN 55672 Teil 1 festgelegt.

### NCO-Gehalt:

Der NCO-Gehalt wurde, wenn nicht ausdrücklich anders erwähnt, volumetrisch gemäß DIN-EN ISO 11909 bestimmt.

### Viskosität:

Die Viskosität wurde nach ISO 3219 bei 23°C bestimmt.

### Restmonomergehalt:

Die Bestimmung des Restmonomergehalts erfolgte nach DIN ISO 17025.

Als NMR wurde ein Bruker DRX 700 Gerät verwendet.

### Synthese der beta-Aminosäureester:

Das primäre Diamin wird unter Stickstoffatmosphäre vorgelegt und das Diethylacrylat unter Rühren zu getropft, so dass das eine Reaktionstemperatur von 30°C nicht überschritten wird. Nach abgeklungener Exothermie wird für 12 Stunden auf 60°C erhitzt.

### Hergestellte beta-Aminosäureester :

| Name | Äquivalent-gewicht (g/mol) | Diacrylat | Menge (g) | Diamin | Menge (g) |
|---|---|---|---|---|---|
| P-b-ae1 | 570,80 | Poly(ethylenglykol)diacrylat (M=575 g/mol) | 148,86 | Jeffamin 600 | 180,00 |
| P-b-ae2 | 524,34 | Poly(ethylenglykol)diacrylat (M=575 g/mol) | 110,36 | Jeffamin 600 | 180,00 |
| P-b-ae3 | 558,89 | Poly(ethylenglykol)diacrylat (M=700 g/mol) | 136,85 | Jeffamin 600 | 180,00 |
| P-b-ae4 | 622,20 | Poly(ethylenglykol)diacrylat (M=700 g/mol) | 178,62 | Jeffamin 600 | 180,00 |
| P-b-ae5 | 378,12 | Poly(ethylenglykol)diacrylat (M=700 g/mol) | 68,00 | Isophorondiamin | 19,90 |
| P-b-ae6 | 457,10 | Poly(ethylenglykol)diacrylat (M=700 g/mol) | 83,40 | Hexamethylendia min | 16,60 |
| P-b-ae7 | 408,75 | Hexandioldiacrylat (Laromer HDDA) | 23,90 | Jeffamin 600 | 76,10 |

### Synthese der Polyester:

A) Aus einer gerührten Mischung bestehend aus 24,7g Adipinsäure und 281,4g PEG 600 wurden über 12h bei 210-220°C bei Atmosphärendruck Wasser abdestilliert. Anschließend wurden 30 mg Zinn(II)chlorid der Mischung zugegeben und unter Vakuum für weitere 24h Wasser abdestilliert, bis das Gemisch eine Säurezahl kleiner als 1 aufwies.
B) Aus einer gerührten Mischung bestehend aus 212,55g Bernsteinsäure und 1591,56g PEG 600 wurden über 12h bei 210-220°C bei Atmosphärendruck Wasser abdestilliert. Anschließend wurden 180 mg Zinn(II)chlorid der Mischung zugegeben und unter Vakuum für weitere 24h Wasser abdestilliert, bis das Gemisch eine Säurezahl kleiner als 1 aufwies.
C) Aus einer gerührten Mischung bestehend aus 49,29g Bernsteinsäure und 519,6g PEG 800 wurden über 12h bei 210-220°C bei Atmosphärendruck Wasser abdestilliert. Anschließend wurden 50 mg Zinn(II)chlorid der Mischung zugegeben und unter Vakuum für weitere 24h Wasser abdestilliert, bis das Gemisch eine Säurezahl kleiner als 1 aufwies.

### Synthese des HDI Präpolymers:

PA) 336 g HDI wurden mit 0,1 Gew% Benzoylchlorid vorgelegt und auf 80 °C erhitzt. Anschließend wurden unter Rühren 197,7 g des zuvor hergestellten Polyesters A) über 1h zudosiert und bis zum Erreichen eines konstanten NCO-Gehalts bei 80 °C weiter gerührt. Das überschüssige HDI wurde bei 140 °C und 0,13 mbar mittels Dünnschichtverdampfer entfernt. Das erhaltene Präpolymer hatte einen NCO-Gehalt von 5,8 % (Äquivalentgewicht: 724,14 g/mol) und eine Viskosität von 1010 mPas/23°C. Der Restmonomergehalt betrug < 0,03 % HDI.

PB) 672 g HDI wurden mit 0,1 Gew% Benzoylchlorid vorgelegt und auf 80 °C erhitzt. Anschließend wurden unter Rühren 788 g des zuvor hergestellten Polyesters B) über 1h zudosiert und bis zum Erreichen eines konstanten NCO-Gehalts bei 80 °C weiter gerührt. Das überschüssige HDI wurde bei 140 °C und 0,13 mbar mittels Dünnschichtverdampfer entfernt. Das erhaltene Präpolymer hatte einen NCO-Gehalt von 3,5 % (Äquivalentgewicht: 1200,00 g/mol) und eine Viskosität von 4800 mPas/23°C. Der Restmonomergehalt betrugt 0,03 % HDI.

PC) 252 g HDI wurden mit 0,1 Gew% Benzoylchlorid vorgelegt und auf 80 °C erhitzt. Anschließend wurden unter Rühren 307,2 g des zuvor hergestellten Polyesters C) über 1h zudosiert und bis zum Erreichen eines konstanten NCO-Gehalts bei 80 °C weiter gerührt. Das überschüssige HDI wurde bei 140 °C und 0,13 mbar mittels Dünnschichtverdampfer entfernt. Das erhaltene Präpolymer hatte einen NCO-Gehalt von 2,8 % (Äquivalentgewicht: 1500,00 g/mol) und eine Viskosität von 6460 mPas/23°C. Der Restmonomergehalt betrugt 0,03 % HDI.

### Herstellung der Hydrogele:

Eine äquivalente Menge eines beta-Aminosäureesters (P-b-ae 1-6) als Härter und eine äquivalente Menge eines HDI-Polyesterpräpolymers (PA, PB, PC) werden gentrennt voneinander in Wasser gelöst (siehe Tabelle) so dass zwei Lösungen entstehen, die ein identisches Volumen aufweisen (Polymergehalt in Summe (beta-Aminosäureester + HDI-Präpolymer) = 10% der Gesamtmasse = (beta-aminosäureester + HDI-Präpolymer + Wasser). Anschließend werden die beiden Lösungen synchron (mit gleichen Volumenströmen) unter Rühren in ein Becherglas gegeben. Innerhalb von 15 - 60 Sekunden bildete sich ein Hydrogel.

Eingewogene Mengen (Angaben in Gramm):

| | Pbae1 | Pbae2 | Pbae3 | Pbae4 | Pbae5 | Pbae6 | Pbae7 |
|---|---|---|---|---|---|---|---|
| Einwaage (P-b-ae-x) | 0,88 | 0,84 | 0,87 | 0,92 | 0,69 | 0,77 | 0,72 |
| Einwaage Wasser | 9,12 | 9,16 | 9,13 | 9,08 | 9,31 | 9,23 | 9,28 |
| Einwage Prepolymer (PA) | 1,12 | 1,16 | 1,13 | 1,08 | 1,31 | 1,23 | 1,28 |
| Einwaage Wasser | 8,88 | 8,84 | 8,87 | 8,92 | 8,69 | 8,77 | 8,72 |
| Einwaage (P-b-ae-x) | 0,64 | 0,61 | 0,64 | 0,68 | 0,48 | 0,55 | 0,51 |
| Einwaage Wasser | 9,36 | 9,39 | 9,36 | 9,32 | 9,52 | 9,45 | 9,49 |
| Einwage Prepolymer (PB) | 1,36 | 1,39 | 1,36 | 1,32 | 1,52 | 1,45 | 1,49 |
| Einwaage Wasser | 8,64 | 8,61 | 8,64 | 8,68 | 8,48 | 8,55 | 8,51 |
| Einwaage (P-b-ae-x) | 0,55 | 0,52 | 0,54 | 0,59 | 0,40 | 0,47 | 0,43 |
| Einwaage Wasser | 9,45 | 9,48 | 9,46 | 9,41 | 9,60 | 9,53 | 9,57 |
| Einwaage Prepolymer (PC) | 1,45 | 1,48 | 1,46 | 1,41 | 1,60 | 1,53 | 1,57 |
| Einwaage Wasser | 8,55 | 8,52 | 8,54 | 8,59 | 8,40 | 8,47 | 8,43 |

### In vitro Abbauversuche der Hydrogele:

Ein zylinderförmiges Stück des Gels mit ca. 5 cm Höhe und 0,6 cm Durchmesser wird bei 37°C in PBS saliner Pufferlösung (pH 7,4, Aldrich P-5368) im Schüttelinkubator gelagert und täglich überprüft, ob sich das Probenstück aufgelöst hat. Eine Probe gilt als abgebaut, wenn kein Festkörper mehr vorhanden ist.

In vitro Abbauversuche mit Hydrogelen (10%-Polymergehalt):

| | P-b-ae 1 | P-b-ae 2 | P-b-ae 3 | P-b-ae 4 | P-b-ae 5 | P-b-ae 6 |
|---|---|---|---|---|---|---|
| PA | 12 d | 2 d | 7 d | 11 d | 20 d | 23 d |
| PB | 6 d | 8 d | 1 d | 6 d | - | - |
| PC | 1 d | 1 d | 1 d | 1 d | 3 d | 5 d |

Die Ergebnisse der Abbauversuche unter simulierten Bedingungen belegen, dass sich die mit dem erfindungsgemäßen beta-Aminosäureester hergestellten Polyharnstoff Hydrogele innerhalb des Körpers in kurzer Zeit abbauen. Zudem ist erkennbar, dass sich durch Wahl der miteinander zum Polyharnstoff umgesetzten Komponenten die Abbaugeschwindigkeit des Hydrogels einstellen lässt.

## Patentansprüche

1. Beta-Aminosäureester erhältlich durch Umsetzung eines Di-Acrylats der allgemeinen Formel (I) 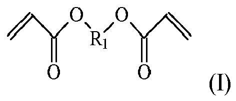 mit einem Diamin der allgemeinen Formel (II) 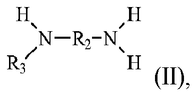 wobei R₁ und R₂ organische Reste ohne Zerewitinoff-aktive H-Atome darstellen und
R₃ für einen organischen Rest ohne Zerewitinoff-aktives H-Atom oder ein Wasserstoffatom steht.

2. Beta-Aminosäureester nach Anspruch 1, **dadurch gekennzeichnet, dass** R₁ und R₂ unabhängig voneinander ausgewählt sind aus Polyesterpolyolgruppen, Polyester-Polyether-Polyolgruppen und/oder Polyetherpolyolgruppen, insbesondere aus Polyester-Polyether-Polyolgruppen und/oder Polyetherpolyolgruppen mit einem Ethylenoxidanteil von wenigstens 60 Gew.-%.

3. Beta-Aminosäureester nach Anspruch 1 oder 2, **dadurch gekennzeichnet, dass** R₁ und R₂ unabhängig voneinander ein Gewichtsmittel von 200 bis 2000 g/mol aufweisen, insbesondere 300 bis 1500 g/mol, bevorzugt 400 bis 1000 g/mol.

4. Beta-Aminosäureester nach einem der vorstehenden Ansprüche, **dadurch gekennzeichnet, dass** R₃ ausgewählt ist aus gesättigten oder ungesättigten Kohlenwasserstoffresten, insbesondere aus Methyl-, Ethyl- oder Propylresten.

5. Beta-Aminosäureester nach einem der vorstehenden Ansprüche, **dadurch gekennzeichnet, dass** der beta-Aminosäureester ein zahlenmittleres Molekulargewicht von 1300 bis 25000 aufweist, insbesondere von 3000 bis 12000.

6. Verfahren zur Herstellung eines beta-Aminosäureesters nach einem der Ansprüche 1 bis 5 bei dem man ein Di-Acrylat der allgemeinen Formel (I) mit einem Diamin der allgemeinen Formel (II) umsetzt.

7. Polyharnstoff-System, umfassend
als Komponente A) isocyanatfunktionelle Präpolymere erhältlich durch Umsetzung von
aliphatischen Polyisocyanaten A1) mit
Polyolen A2), die insbesondere ein zahlenmittleres Molekulargewicht von ≥ 400 g/mol und eine mittlere OH-Funktionalität von 2 bis 6 aufweisen können,
als Komponente B) einen Beta-Aminosäureester gemäß einem der Ansprüche 1 bis 5,
gegebenenfalls als Komponente C) organische Füllstoffe, die insbesondere eine nach DIN 53019 gemessenen Viskosität bei 23 °C im Bereich von 10 bis 6000 mPas aufweisen können,
gegebenenfalls als Komponente D) Umsetzungsprodukte von isocyanatfunktionellen Präpolymeren gemäß Komponente A) mit einem Beta-Aminosäureester gemäß Komponente B) und/oder organischen Füllstoffen gemäß Komponente C) und gegebenenfalls als Komponente E) Wasser und/oder ein tertiäres Amin.

8. Polyharnstoff System nach Anspruch 7, **dadurch gekennzeichnet, dass** die Polyole A2) Polyesterpolyole und/oder Polyester-Polyether-Polyole und/oder Polyetherpolyole, insbesondere Polyester-Polyether-Polyole und/oder Polyetherpolyole mit einem Ethylenoxidanteil zwischen 60 und 90 Gew.-%, enthalten.

9. Polyharnstoff System nach einem der Ansprüche 7 oder 8, **dadurch gekennzeichnet, dass** die Polyole A2) ein zahlenmittleres Molekulargewicht von 4000 bis 8500 g/mol aufweisen.

10. Polyharnstoff System nach einem der Ansprüche 7 bis 9, **dadurch gekennzeichnet, dass** die Präpolymere A) eine mittlere NCO-Funktionalität von 1,5 bis 2,5, bevorzugt von 1,6 bis 2,4, weiter bevorzugt von 1,7 bis 2,3, ganz besonders bevorzugt von 1,8 bis 2,2 und insbesondere von 2 aufweisen.

11. Polyharnstoff System nach einem der Ansprüche 7 bis 10, **dadurch gekennzeichnet, dass** es sich bei den organischen Füllstoffen der Komponente C) um hydroxyfunktionelle Verbindungen, insbesondere um Polyetherpolyole mit sich wiederholenden Ethylenoxid Einheiten handelt.

12. Polyharnstoff-System nach Anspruch 11, **dadurch gekennzeichnet, dass** die Füllstoffe der Komponente C) eine mittlere OH-Funktionalität von 1,5 bis 3, bevorzugt von 1,8 bis 2,2 und besonders bevorzugt von 2 aufweisen.

13. Polyharnstoff System nach einem der Ansprüche 7 bis 12, **dadurch gekennzeichnet, dass** die Komponente E) ein tertiäres Amin der allgemeinen Formel (V) 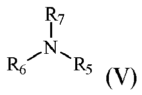 enthält, in der
R₅, R₆, R₇ unabhängig voneinander Alkyl- oder Heteroalkyreste mit Heteroatomen in der Alkylkette oder an deren Ende sein können, oder R₅ und R₆ gemeinsam mit dem sie tragenden Stickstoffatom einen aliphatischen, ungesättigten oder aromatischen Heterozyklus bilden können, der gegebenenfalls weitere Heteroatome enthalten kann.

14. Polyharnstoff System nach einem der Ansprüche 7 bis 13, **dadurch gekennzeichnet, dass** das tertiäre Amin ausgewählt aus der Gruppe Triethanolamin, Tetrakis (2-hydroxyethyl) ethylendiamin, N,N-Dimethyl-2-(4-methylpiperazin-1-yl)ethanamin, 2-{[2-(Dimethylamino)ethyl](methyl)amino}ethanol, 3,3',3"-(1,3,5-Triazinan-1,3,5-triyl)tris(N,N-dimethyl-propan-1-amin) ist.

15. Polyharnstoff System nach einem der Ansprüche 7 bis 14, **dadurch gekennzeichnet, dass** Komponente E) 0,2 bis 95 Gew.-% Wasser und/oder 0,1 bis 1,0 Gew.-% des tertiäres Amin bezogen auf das Polyharnstoff System enthält, insbesondere 10 bis 95 Gew.-% Wasser.

16. Polyhamstoff-System nach einem der Ansprüche 7 bis 15, **dadurch gekennzeichnet, dass** ferner wenigstens ein pharmakologisch aktiver Wirkstoff enthalten ist, der insbesondere ausgewählt ist aus Analgetika mit und ohne antiinflammatorische Wirkung, Antiphlogistika, antimikrobiell wirksame Substanzen, Antimykotika, antiparasitär wirkenden Stoffen oder Kombinationen hiervon.

17. Polyharnstoff System nach einem der Ansprüche 7 bis 16 zum Verschließen, Verbinden, Verkleben oder Abdecken von Zellgewebe, insbesondere zur Erzeugung einer Adhäsionsbarriere, zur Stillung des Austritts von Blut oder Gewebeflüssigkeiten oder dem Verschluss von Leckagen in Zellgewebe.

18. Polyharnstoff System nach Anspruch 17 zur Verwendung zum Verschließen, Verbinden, Verkleben oder Abdecken von menschliches oder tierisches Zellgewebe.

19. Dosiersystem mit zwei Kammern für ein Polyharnstoff System nach einem der Ansprüche 7 bis 18, **dadurch gekennzeichnet, dass** in der einen Kammer die Komponente A) und eine erste Teilmenge von E) in der anderen Kammer die Komponenten B) und gegebenenfalls die Komponenten C), D) und eine zweite Teilmenge von E) des Polyharnstoff Systems enthalten sind.

20. Dosiersystem gemäß Anspruch 19, **dadurch gekennzeichnet, dass** die Komponenten A) und B) in zueinander äquivalenten Stoffmengen vorhanden sind und die erste und zweite Teilmenge von E) jeweils so gewählt ist, dass das Volumen von A) und erster Teilmenge von E) weitestgehend identisch zum Volumen aus B), der zweiten Teilmenge von E) und der optionalen Komponenten C) und D) ist.
